# EUROPEAN PATENT SPECIFICATION

(11) **EP 1 255 870 B1**
(45) Date of publication and mention of the grant of the patent: **14.12.2005**
(21) Application number: 01916089.4
(22) Date of filing: 14.02.2001
(51) Int. Cl.: C12Q 1/68, C07K 14/705, A61K 31/415

(54) **5-HYDROXYTRYPTAMINE TRANSPORTER GENE POLYMORPHISMS**
POLYMORPHISMEN DES 5-HYDROXYTRYPTAMINTRANSPORTERGENS
POLYMORPHISMES DE GENE TRANSPORTEUR DE 5-HYDROXYTRYPTAMINE

(30) Priority: 17.02.2000 US 183255 P; 05.04.2000 US 194956 P
(43) Date of publication of application: 13.11.2002
(73) Proprietor: GLAXO GROUP LIMITED, Greenford, Middlesex UB6 ONN (GB)
(72) Inventor: KONG, Ning, Steven, Groton, MA 01450 (US); MANASCO, Penelope, K., c/o Glaxo Wellcome Inc., Research Triangle Park, NC 27709 (US); MOSTELLER, Michael, Jr., c/o GlaxoSmithKline, Research Triangle Park, NC 27709 (US)
(74) Representative: Giddings, Peter John
(86) International application number: PCT/US2001/004755
(87) International publication number: WO 2001/061039

(56) References cited:
- WO-A-01/77388
- WO-A-94/09828
- VINCENT JOHN B ET AL: "Genetic association analysis of serotonin system genes in bipolar affective disorder." AMERICAN JOURNAL OF PSYCHIATRY, vol. 156, no. 1, January 1999 (1999-01), pages 136-138, XP002938287 ISSN: 0002-953X

## Description

### Field of the Invention

The present studies relate to polymorphisms in the 5-hydroxytryptamine transporter (5-HTT) gene, and phenotypes that are associated or correlated therewith. More particularly, the present studies relate to the correlation of such polymorphisms to the response of subjects with gastrointestinal disorders (such as Irritable Bowel Syndrome (IBS)) to pharmaceutical treatment. The present studies further relate to methods of screening compounds for pharmaceutical activity. The present studies also relate to methods of genotyping subjects for predictive purposes.

### Background of the Invention

Many gastrointestinal disorders of unknown etiology, including Irritable Bowel Syndrome (IBS), are believed to be multifactorial disorders. In many of these disorders, no biochemical marker has been found and diagnosis is accomplished primarily by observation of clinical symptoms. Unlike single gene Mendelian disorders, complex disorders such as diabetes, migraine and cardiovascular disease tend to be multifactorial and are caused by the interaction of one or more susceptibility genes with environmental factors. To date, no individual susceptibility genes for IBS have been identified by either linkage or association studies.

Irritable bowel syndrome (IBS) is a common gastrointestinal disorder characterized by abdominal pain and discomfort, and altered bowel habit. IBS may be characterized by altered bowel habit symptoms of either constipation or diarrhea, or alternating constipation and diarrhea. Currently, there is no single pathophysiological or diagnostic marker of IBS. However, various diagnostic criteria for IBS are available, e.g.., Thompson et al., Gastroent. Int., 2:92 (1989); Manning et al., Br. Med. J. 2:653 (1978); Thompson et al., Gut 45:1143 (1999)
Antagonism at 5-hydroxytryptamine receptors, such as by alosetron hydrochloride, has been shown to be useful in the treatment of diarrhea-predominant irritable bowel syndrome.

Alosetron hydrochloride (CAS registry number: CAS-122852-69-1; see US Patent No. 5,360,800) is a 5-HT3 receptor antagonist. Both animal and human studies indicate that 5-HT3 receptor blockade has therapeutic value in the treatment of irritable bowel syndrome, particularly in diarrhea-predominant IBS. (The disclosures of all US patents cited herein are incorporated herein by reference in their entirety.)

In double-blind, placebo controlled studies, alosetron hydrochloride has been shown to reduce pain and improve bowel function in patients with Irritable Bowel Syndrome (IBS). *See* Bardhan et al., Aliment Pharmacol Ther 2000 Jan;14(1):23-34; Jones et al., Aliment Pharmacol Ther 1999 Nov;13(11):1419-27; Camilleri et al., Aliment Pharmacol Ther 1999 Sep;13(9):1149-59; Mangel et al., Aliment Pharmacol Ther 1999 May;13 Suppl 2:77-82. Alosetron has further been indicated as a potential treatment for the symptomatic relief of carcinoid diarrhea. Saslow et al., Gut 1998 May;42(5):628-34.

5-hydroxytryptamine (5HT) receptors have been identified and characterized in the gastrointestinal tract, including 5HT3, 5HT4, and 5HT1a receptors; these receptors are involved not only in modulating gut motility but also in visceral sensory pathways. Various 5HT3 antagonists (e.g., alosetron, granisetron and ondansetron) have been identified for the treatment of IBS. This class of drug appears to reduce visceral sensitivity and have inhibitory effects on motor activity in the distal intestine. Full and partial 5HT4 agonists (e.g., HTF919, tegaserod) are potential therapeutics to improve constipation-predominant IBS. Preliminary studies suggest that these agents may have therapeutic potential in IBS. Farthing et al., Baillieres Best Pract Res Clin Gastroenterol. 1999 Oct;13(3):461-71. 5HT4 antagonists (piboserod, SB-207266A) have also been suggested for the treatment of IBS.

The human 5HTT is encoded by a single gene (SLC6A4) found on chromosome 17q 12 (Ramamoorthy et al., Proc. Natl. Acad. Sci. USA 90:2542 (1993); Gelernter et al., Hum. Genet. 95:677 (1995); Lesch et al., J. Neural Transm. 91:67 (1993). The 5HT transporter regulates the magnitude and duration of serotonergic responses. An insertion/deletion polymorphism consisting of a 44 base pair segment in the transcriptional control region 5' upstream to the 5HTT coding sequence has previously been identified. The deletion (or short) allele of this polymorphism is associated with decreased transcription efficiency of the 5HTT gene promoter, decreased gene expression, and decreased 5-hydroxytryptamine uptake. (Heils et al., J. Neural Transm. 102:247 (1995); Heils et al., J. Neurochem 66:2621 (1996), Lesch et al., Science 274:1527 (1996)). Various biochemical studies suggest that 5HT uptake function is frequently reduced in psychiatric illnesses, and variation in functional 5HTT expression due to 5HTT promoter polymorphism has been implicated as a potential genetic susceptibility factor for affective disorders (Collier et al., Mol Psychiatry 1996 Dec;1 (6):453-60; Lesch et al., Science 1996 Nov 29;274(5292):1527-31; Furlong et al., Am J Med Genet 1998 Feb 7;81(1):58-63; Menza et al., J Geriatr Psychiatry Neurol 1999 Summer; 12(2):49-52; and Rosenthal et al., Mol Psychiatry 1998 Mar;3(2):175-7.

WO94/09828 discloses an isolated nucleic acid molecule encoding a mammalian 5-HT_{4B} receptor. The protein may be used in methods for determining ligand binding, receptor expression, drug screening and in treatments for alleviating abnormalities associated with the receptor such as irritable bowel syndrome.

In Vincent J.B. et al, American Journal of Psychiatry, 156:1, (Jan 1999), p.136-138, a genetic association analysis found statistically significant positive association between bipolar affective disorder and polymorphisms and of human serotonin gene (HTR2A) and the serotonin transporter (hSert).

### Summary of the Invention

The present inventors have determined that polymorphisms in the 5-hydroxytryptamine transporter (5HTT) gene are correlated with the response of subjects with IBS to pharmaceutical therapy. More particularly, they have found that an insertion/deletion polymorphism in the 5' non-coding region of the 5HTT gene is a predictor for the response of patients with IBS to treatment with a 5HT antagonist; and have identified a genetic subset of IBS patients that displays a higher incidence of relief of IBS symptoms and a lower incidence of constipation when treated with alosetron (compared to patients with an alternative polymorphism at the same site of the 5HTT gene).

A first aspect of the present invention is a method of screening a patient population to identify those subjects with an increased likelihood of responding favorably to treatment with a 5HT antagonist for a gastrointestinal disorder. The subjects may have been previously diagnosed as having IBS or another gastrointestinal disorder treatable with a 5HT receptor ligand, or the screening may be used in conjunction with IBS diagnostic efforts.

A further aspect of the present invention is a method of screening a subject suffering from a gastrointestinal disease that is treatable with a 5-hydroxytryptamine (5HT) ligand, as an aid in predicting the subject's response to treatment with a 5HT ligand. The method comprises obtaining a sample of the subject's DNA and determining the genotype of the subject at a polymorphic allelic site in the 5hydroxytryptamine transporter (5HTT) gene, where different genotypes at that site have been associated with different incidences of a phenotypic response to treatment with a 5HT ligand. The genotype that is detected in the sample indicates that the subject is likely to have the phenotypic response associated with that genotype.

A further aspect of the present invention is a method of screening a subject with irritable bowel syndrome (IBS), as an aid in predicting the subject's response to treatment with a 5HT ligand. The method comprises obtaining a sample of the subject's DNA and determining the genotype of the subject at a polymorphic allelic site in the 5hydroxytryptamine transporter (5HTT) gene, where different genotypes at that site have been associated with different incidences of a phenotypic response to treatment with a 5HT ligand.

A further aspect of the present invention is a method of screening a 5-hydroxytryptamine (5HT) ligand for variations in a measurable phenotypic effect among genetic subpopulations of subjects with a gastrointestinal disorder. The method comprises administering the 5HT ligand to a population of subjects suffering from the gastrointestinal disorder, and obtaining DNA samples from each of the subjects. The DNA samples are genotypes for a polymorphic allele of the 5-hydroxytryptamine transporter (5HTT) gene, and correlations between the polymorphic allele genotype and the occurrence of a phenotypic response in the population of subjects are determined. Detection of a genotype that is correlated with an increased or decreased incidence of a desired therapeutic response or a side effect (compared to the incidence in subjects with alternative genotypes) indicates that the effectiveness of the ligand in treating that gastrointestinal disorder varies among genetic subpopulations.

A further aspect of the present invention is a method of treating subjects with Irritable Bowel Syndrome (IBS) by administering a 5HT3 receptor antagonist, where the patients have a polymorphism in the 5HTT gene that is predictive of a higher incidence of relief of IBS symptoms or a lower incidence of side effects when treated with a 5HT3 receptor antagonist. The incidence of relief is increased (and of side effects decreased) compared to subjects who have an alternative polymorphism at the same site of the 5HTT gene.

A further aspect of the present invention is a method of treating subjects with Irritable Bowel Syndrome (IBS) by administering alosetron to the subjects, where they have a polymorphism in the 5HTT gene that is predictive of a higher incidence of relief of IBS symptoms and a lower incidence of constipation when treated with alosetron (compared to subjects with an alternative polymorphism at the same site of the 5HTT gene).

A further aspect of the present invention is a method of treating a subject suffering from Irritable Bowel Syndrome (IBS), by identifying the genotype of the subject at a polymorphic allelic site in the 5hydroxytryptamine transporter (5HTT) gene, where different genotypes at this site are associated with different incidences of phenotypic response to treatment of IBS with a 5HT receptor ligand. The subject is administered a 5HT receptor ligand that is associated with an increased incidence of a favorable phenotypic response in subjects with the identified genotype.

### Brief Description of the Figures

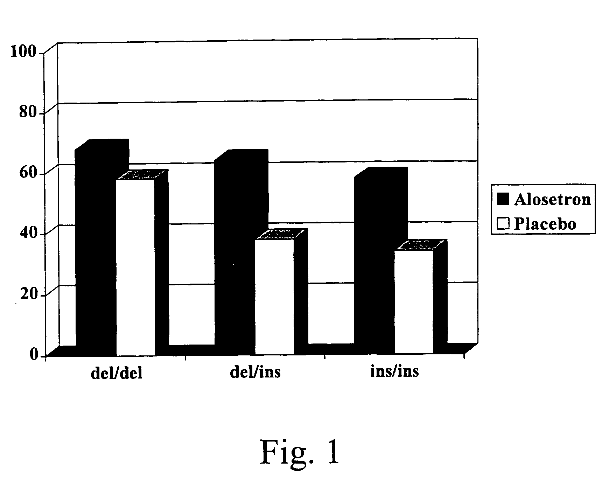

**Figure 1** is a graph based on data collected from female patients enrolled in clinical trials of alosetron for the treatment of IBS. The study population was comprised of non-constipated individuals with IBS. The subjects were divided into 5HTT genotypes. Of the 219 subjects, 71 (32.4%) were del/del 5HTT, 75 (34.2%) were del/ins 5HTT and 73 (33.3%) were ins/ins 5HTT. **Figure 1** shows the percentage of subjects that responded to treatment with either alosetron or a placebo, divided into 5HTT genotypes. "Response'' was defined as relief of IBS symptoms (abdominal pain and discomfort) during six weeks of a twelve week treatment study. Two hundred and nineteen subjects, received either alosetron (102 subjects) or matched placebo (117 subjects). The proportion of patients achieving response following treatment with alosetron was 68% (21/31) for del/del 5HTT, 64% (21/33) for del/ins 5HTT and 58% (22/38) for ins/ins 5HTT. The proportion of patients achieving response following treatment with placebo was 58% (23/40) for del/del 5HTT, 38% (16/42) for del/ins 5HTT and 34% (12/35) for ins/ins 5HTT.

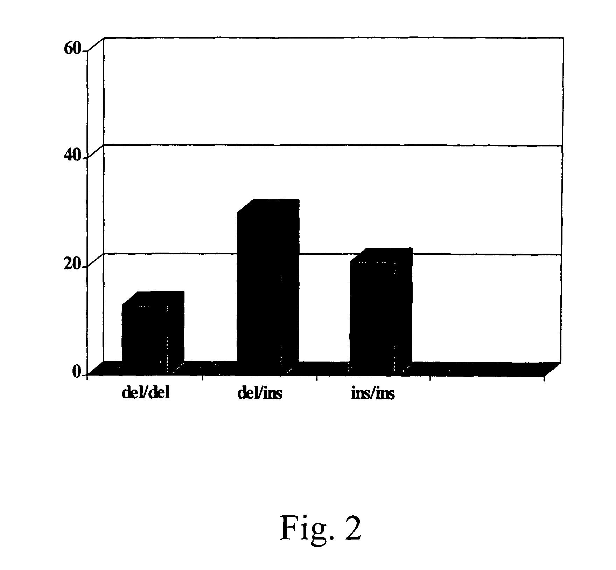

**Figure 2** compares, among 5HTT genotypes, the percentage of alosetron-treated subjects who reported constipation. Of the 102 alosetron treated subjects, the proportion of subjects reporting constipation was 13% (4/31) for del/del 5HTT, 30% (10133) for dellins 5HTT and 21% (8/38) for ins/ins 5HTT.

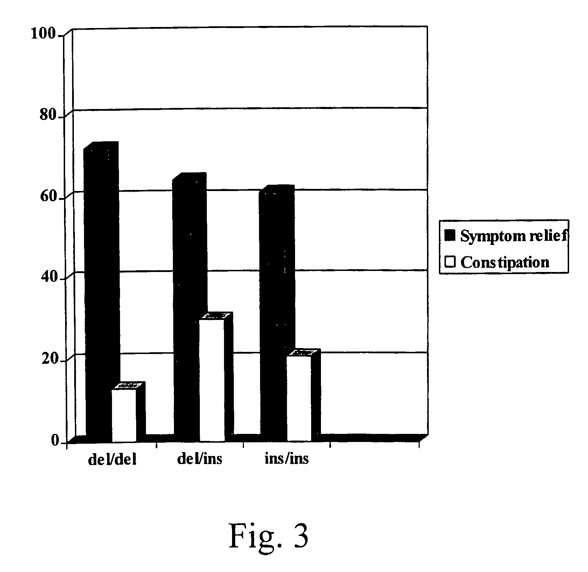

**Figure 3** is a composite of the information shown in Figures 1 and 2, and compares among 5HTT genotypes the incidence of constipation and the percentage of subjects experiencing relief of IBS symptoms, in alosetron-treated subjects. Subjects with the del/del 5HTT genotype showed an increased incidence of favourable therapeutic response with a higher incidence of relief of IBS symptoms and a lower incidence of constipation, when compared with subjects who had del/ins or ins/ins 5HTT genotypes.

### Detailed Description of the Invention

The present invention is concerned with the treatment of gastrointestinal disorders mediated by 5HT receptors, more particularly with the treatment of Irritable Bowel Syndrome (IBS), and more particularly with the treatment of non-constipation-predominant IBS. The present inventors have determined that polymorphic variations in the 5HTT gene can be correlated to, or associated with, the response to pharmaceutical treatment, particularly treatment with 5HT receptor ligands and more particularly treatment with 5HT3 antagonists. The present inventors have identified that an insertion/deletion polymorphism in the 5' untranslated region of the 5HT transporter (5HTT) gene is correlated with the response of subjects with a gastrointestinal disorder to treatment with a 5HT ligand.

Genetic samples were obtained from subjects enrolled in clinical trials of alosetron for the treatment of IBS. The genetic samples were screened for an insertion/deletion polymorphism in the 5' non-coding region of the 5-hydroxytryptamine transporter gene (5HTT gene), using polymerase chain reaction (PCR) technology. The alleles were labeled as "del" (deletion) or "ins" (insertion) resulting in three possible genotypes (del/del; del/ins or ins/ins). The insertion polymorphism (allele "ins") had SEQ ID NO:2:
- Legend:: PCR primer sequences are in underlined typeface Non-coding sequences are shown in lowercase typeface Polymorphic bases are shown in bold typeface Base numbering is relative to the sequence shown Polymorphism numbering is relative to the gene cDNA sequences

The "del" allele represents a deletion of approximately 44 base pairs in the 5' untranslated region of the 5HTT gene. This deletion in the transcriptional regulatory region has been associated with decreased re-uptake of 5HT and therefore an increased 5HT basal level. Therefore, the del/del genotype is postulated to result in a lower transcription efficiency, lower production of 5HTT, and reduced basal 5HT re-uptake (compared to the del/ins or ins/ins genotype). The del/del, del/ins and ins/ins genotypes were approximately evenly distributed among the subjects. Of 219 subjects, 71 were del/del 5HTT; 75 were del/ins 5HTT; and 73 were ins/ins 5HTT.

The present inventors determined that the del/del genotype is associated with an increased incidence of relief of IBS symptoms and a lower frequency of constipation as an effect of treatment with a 5HT3 antagonist, and therefore an increased incidence of favorable therapeutic response to treatment with a 5HT3 antagonist (compared to subjects with the del/ins or ins/ins genotype treated with the same 5HT3 antagonist).

In each of the three 5HTT genotypes alosetron was more effective than placebo in relieving IBS symptoms (**Figure 1**). However, in the del/del genotype group (homozygous for the deletion polymorphism), the incidence of relief of IBS symptoms for both alosetron and placebo was increased compared to other 5HTT genotypes (**Figure 1**). Subjects with the del/del genotype also showed a reduced incidence of constipation compared to the del/ins and ins/ins 5HTT genotype groups (**Figure 2**). **Figure 3** shows subjects with the del/del 5HTT genotype showed an increased incidence of favourable therapeutic response with a higher incidence of relief of IBS symptoms and a lower incidence of constipation, when compared with subjects who had del/ins or ins/ins 5HTT genotypes.

Additional polymorphisms have been identified in the 5HTT gene that may be useful as indicators of phenotypic response to treatment with a 5HT ligand for gastrointestinal disease. These include seven Single Nucleotide Polymorphisms (SNPs) identified by searching for SNPs within DNA samples from 30 test individuals. The 5HTT gene was amplified and PCR products were sequenced to identify SNPs. Seven SNPs (see Table 1) with a minor allele frequency of >5% (within the 30-person population) were further tested.

| SNP | GenBank Accession Number | Polymorphism; Sequence Position |
|---|---|---|
| T623C | X76753 | change from T toC; at bp 623 |
| T3287C | X76753 | change from T to C; at bp3287 |
| G674A | U79746 | change from G to A; at bp674 |
| C867T | U79746 | change from C to T; at bp 867 |
| A2631C | U79746 | change from A to C; at bp2631 |
| G160A | X76758 | change from G to A; at bp 160 |
| G769T | X76762 | change from G to T; at bp769 |

The SNPs are identified in Table 1 by the change in nucleotide and the position of the polymorphism; the numbering of nucleotides is that of the corresponding GenBank sequence.

A further polymorphism identified and screened in the 5HTT gene is a Variable Number Tandem Repeat (VNTR) polymorphism found in intron 2 of the 5HTT gene, consisting of multiple repeats of a 17-basepair sequence (see bp 843-1012 in GenBank Accession Number X76754). A common genotype consists of ten copies of the 17-bp repeat sequence, but the number of 17 basepair repeats varies, and may be from fewer than nine, from 9 to 12, to more than twelve repeats.

After the initial identification of these SNPs and the VNTR polymorphism, genetic samples were obtained from subjects enrolled in clinical trials of alosetron for the treatment of IBS, including a population of subjects with non-constipation predominant IBS. The genetic samples are screened for the identified polymorphisms, using polymerase chain reaction (PCR) technology as is known in the art. The occurrence of a particular genotype is correlated to the subjects' phenotypic response to treatment with alosetron; such phenotypic responses include achieving adequate response to treatment, incidence of side effects, and time course of response to treatment.

According to the present methods, a subject who suffers from a gastrointestinal disease that is treatable with 5HT ligands can be genetically screened, to aid in predicting their response to such treatment. Screening comprises obtaining a sample of DNA from the subject and screening the DNA to determine the genotype (presence/absence of polymorphic alleles) at a predetermined polymorphic site in the 5hydroxytryptamine transporter (5HTT) gene, where different genotypes at that site have previously been associated with different incidences of a phenotypic response to treatment with a 5HT ligand. The presence of a particular genotype therefore indicates an increased likelihood that the individual subject will exhibit the associated phenotype. The genotype will rarely be absolutely predictive, i.e., where a population with a certain genotype displays a high incidence of a particular phenotype, not every individual with that genotype will display the phenotype. However, it will be apparent to those skilled in the art that genotyping a subject as described herein will be an aid in predicting the response a subject will have to treatment with a 5HT ligand, and thus assist in the treatment decision.

As used herein, "genotyping a subject (or DNA sample) for a polymorphic allele at a defined genomic locus" or "determining the genotype at a polymorphic allelic site" means detecting which forms of the allele are present in a subject (or a sample). As is well known in the art, an individual may be heterozygous or homozygous for a particular allele. More than two forms of an allele may exist, as is the case with microsatellite markers; thus there may be more than three possible genotypes.

As used herein, a subject that is "predisposed to" a particular phenotypic response based on genotyping of a polymorphic allele will be more likely to display that phenotype than an individual with a different genotype at that polymorphic allele. Where the phenotypic response is based on a biallelic polymorphism, the response may differ among the three possible genotypes (Eg. For 5HTT: del/del, del/ins and ins/ins).

As used herein, a "genetic subset" of a population consists of those members of the population having a particular genotype. In the case of a biallelic polymorphism, a population can potentially be divided into three subsets: homozygous for allele 1, heterozygous, and homozygous for allele 2.

As used herein, a gastrointestinal disease 'treatable with 5HT ligands' is one in which the administration of a 5HT ligand (in an appropriate pharmaceutical formulation, and in a therapeutically effective amount) has been shown to reduce or alleviate symptoms, without causing unacceptable side effects. Such therapeutic effectiveness is typically evidenced by Regulatory Authority (eg FDA, EMEA) approval of the pharmaceutical preparation, or by publication of the results of clinical studies in peer-reviewed medical journals. Therapeutically effective amounts of such compounds can be readily determined by those skilled in the art using, e.g., dose-response studies. As used herein, the term '5HT ligand' encompasses antagonists and agonists of 5HT receptors, including partial agonists and drugs that interact with 5HTT (eg selective serotonin re-uptake inhibitors, SSRI's). 5HT ligands may bind to any subtype of the 5HT receptor, including 5HT3 and 5HT4 receptors; the ligands may be specific for a particular receptor subtype.

Known 5HT-related compounds include 5HT3 antagonists (e.g., ondansetron, granisetron, tropisetron, dolasetron, mirtazapine, itasetron, pancopride, zatosetron, azasetron, cliansetron, YM-144 (Yamanouchi) and RS 17017 (Roche)).

5HT4 agonists are also known, including tegaserod, prucalopride, norcisapride and the 4-amino-5-chloro-2-methoxy-N-(1-substituted piperidin-4-yl)benzamide known as Y-34959 (Yoshitomi Pharmaceuticals), and buspirone. The use of 5HT4 agonists to treat constipation-predominant IBS has been proposed. 5HT4 antagonists include piboserod (SmithKline Beecham).

Dual 5HT3 and 5HT4 agonists include renzapride (SmithKline Beecham) and E3620 (Eisai). A 5HT1a agonist is also known, LY315535 (Eli Lilly).

Selective serotonin re-uptake inhibitors include fluoxetine, etc.

As used herein, a "side effect" is an undesirable response to the administration of a therapeutic compound, i.e., an effect that is not directed to alleviating the symptoms or cause of the disease being treated. Side effects range from minor inconveniences to more serious events.

As used herein, a "favorable" phenotypic response to treatment is a response in which adequate relief of symptoms (e.g., pain, urgency, altered bowel habit) is achieved with an acceptable level of side effects. A particular phenotypic response may be more favorable (e.g., achieve more extensive reduction of symptoms) than another.

Additionally, the phenotypic response may be the average time until adequate relief of symptoms is obtained; particular genotypes are associated with an "early response" phenotype where relief or reduction in symptoms occurs at an earlier time after treatment is initiated, compared to that seen for subjects with alternate genotypes. The desired therapeutic response may be adequate relief of symptoms within, for example, the first month, six weeks, or twelve weeks, compared to subjects in whom adequate relief is not obtained on average until more than 12 weeks, 18 weeks, 24 weeks or longer.

According to the present methods, a compound with 5HT ligand activity may be screened for variation in its effects among genetic subpopulations of subjects with a gastrointestinal disorder. Such methods involve administering the compound to a population of subjects suffering from a 5HT-mediated gastrointestinal disorder, obtaining DNA samples from the subjects (which may be done either prior to or after administration of the compound), genotyping a polymorphic allelic site in the 5HTT gene, and correlating the genotype of the subjects with their phenotypic responses (both favorable and unfavorable) to the treatment. A genotype that is correlated with an increased incidence of a desired therapeutic response (or a decreased incidence of an undesirable side effect), compared to the incidence in subjects with alternative genotypes at the polymorphic allelic site, indicates that the effectiveness of the compound in treating such gastrointestinal disorder varies among genetic subpopulations.

Stated another way, the methods of the present invention may be used to determine the correlation of a known 5HTT polymorphic allele with the response of subjects with gastrointestinal disorders (such as IBS) to treatment with a 5HT ligand. The population of subjects with the disease of interest is stratified according to genotype for the particular polymorphic allele, and their response to a therapeutic agent is assessed (either prospectively or retrospectively) and compared among the genotypes. The response to the therapeutic agent may include either, or both, desired therapeutic responses (e.g., the alleviation of signs or symptoms) and undesirable side effects. In this way, genotypes that are associated with an increased (or decreased) incidence of therapeutic efficacy, or an increased (or decreased) incidence of a particular side effect, may be identified. The increase or decrease in response is in comparison to the other genotypes, or to a population as a whole.

Polymorphisms are variant sequences within the human genome that may or may not have a functional consequence. These variants can be used in all aspects of genetic investigation including the analysis and diagnosis of genetic disease, forensics, evolutionary and population studies. Two types of genetic analyses are typically performed: linkage and association studies.

A linkage study provides genetic map information with no prior knowledge or assumption about the function of a gene. In a linkage study one uses DNA polymorphisms to identify chromosomal regions that are identical between affected relatives with the expectation that allele sharing frequencies will be higher for a marker (polymorphism) whose chromosomal location is close to that of the disease allele. Physical cloning of a linkage region narrows down the DNA sequence that could harbor the candidate disease gene. While linkage analysis locates the disease locus to a specific chromosome or chromosome region, the region of DNA in which to search for the gene is typically large, on the order of several million base pairs.

In contrast to linkage, association shows the coexistence of a polymorphism and a phenotype in a population. Association studies are based upon linkage disequilibrium, a phenomenon that occurs between a marker and a phenotype if the marker polymorphism is situated in close proximity to the functional polymorphism. Since the marker and functional polymorphism are in close proximity, it requires many generations of recombination to separate them in a population. Thus they tend to co-exist together on the same chromosome at a higher than expected frequency. A marker is said to be associated with a specific phenotype when its frequency is significantly higher among one phenotype group compared to its frequency in another. In general, the closer a marker is to the functionally polymorphic site, the stronger the association.

Association studies offer the opportunity to finely map linkage regions, map loci refractory to linkage analysis and map unknown predisposition loci. Polymorphisms that are in linkage disequilibrium with each other can be spaced over large regions. Linkage disequilibrium has been reported in regions as small as 1kb or as large as 500 kb. Polymorphisms throughout a gene can be in linkage disequilibrium with each other, such that it is valuable to study the whole genome structure - introns, exons, promoters and transcriptional regulatory regions, and 3' and 5' untranslated regions. A marker that is in linkage disequilibrium with a functional polymorphism can be tested for correlation with a phenotype.

Polymorphisms useful in the present methods may be either markers in linkage disequilibrium with identified or unidentified functional polymorphisms, or may be functional polymorphisms.
The present inventors have determined that a polymorphism in the 5HTT gene plays a role in the response of subjects to pharmaceutical treatment of IBS, thus the genotyping of the 5HT Transporter (5HTT) gene (either directly or via its expression product) will be useful in identifying therapeutic compounds with measurable effects that vary among 5HTT genotypes. The effect to be measured will depend on the particular gastrointestinal condition, therapeutic compound, and patient population, as will be apparent to one skilled in the art. The measurable effect may be the relief of, or change in, a pathologic sign or symptom or the occurrence of a side effect related to compound administration. Measurement may be objective or subjective (e.g., by patient self-reporting). The association of a 5HTT genotype with a therapeutic response will provide a method of determining the probability that an individual subject will respond in a particular way to treatment with 5HT ligands. In genotyping, the characteristic that is typically measured is one that can be influenced by a polymorphism in the gene or its expression product. As used herein, the term polymorphism includes Single Nucleotide Polymorphisms (SNPs), insertion/deletion polymorphisms; microsatellite polymorphisms; and variable number of tandem repeat (VNTR) polymorphisms.

Polymorphic alleles are typically detected by directly determining the presence of the polymorphic sequence in a polynucleotide or protein from the subject, using any suitable technique as is known in the art. Such a polynucleotide is typically genomic DNA, or a polynucleotide derived from this polynucleotide, such as in a library made using genomic material from the individual (e.g. a cDNA library). The processing of the polynucleotide or protein before the carrying out of the method of the invention is further discussed below. Typically the presence of the polymorphism is determined in a method that comprises contacting a polynucleotide or protein of the individual with a specific binding agent for the polymorphism and determining whether the agent binds to the polynucleotide or protein, where the binding indicates that the polymorphism is present. The binding agent may also bind to flanking nucleotides and amino acids on one or both sides of the polymorphism, for example at least 2, 5, 10, 15 or more flanking nucleotide or amino acids in total or on each side. In one embodiment the agent is able to bind the corresponding wild-type sequence by binding the nucleotides or amino acids which flank the polymorphism position, although the manner of binding will be different than the binding of a polymorphic polynucleotide or protein, and this difference will be detectable (for example this may occur in sequence specific PCR as discussed below).

In the case where the presence of the polymorphism is being determined in a polynucleotide it may be detected in the double stranded form, but is typically detected in the single stranded form.

The binding agent may be a polynucleotide (single or double stranded) typically with a length of at least 10 nucleotides, for example at least 15, 20, 30, or more polynucleotides. The agent may be a molecule that is structurally similar polynucleotides that comprises units (such as purines or pyrimidines) able to participate in Watson-Crick base pairing. The agent may be a protein, typically with a length of at least 10 amino acids, such as at least 20, 30, 50, 100 amino acids. The agent may be an antibody (including a fragment of such an antibody that is capable of binding the polymorphism).

A polynucleotide agent which is used in the method will generally bind to the polymorphism of interest, and the flanking sequence, in a sequence specific manner (e.g. hybridize in accordance with Watson-Crick base pairing) and thus typically has a sequence which is fully or partially complementary to the sequence of the polymorphism and flanking region.

In one embodiment of the present methods a binding agent is used as a probe. The probe may be labeled or may be capable of being labeled indirectly. The detection of the label may be used to detect the presence of the probe on (and hence bound to) the polynucleotide or protein of the individual. The binding of the probe to the polynucleotide or protein may be used to immobilize either the probe or the polynucleotide or protein (and thus to separate it from one composition or solution).

In another embodiment of the invention the polynucleotide or protein of the individual is immobilized on a solid support and then contacted with the probe. The presence of the probe immobilized to the solid support (via its binding to the polymorphism) is then detected, either directly by detecting a label on the probe or indirectly by contacting the probe with a moiety that binds the probe. In the case of detecting a polynucleotide polymorphism the solid support is generally made of nitrocellulose or nylon. In the case of a protein polymorphism the method may be based on an ELISA system.

The present methods may be based on an oligonucleotide ligation assay in which two oligonucleotide probes are used. These probes bind to adjacent areas on the polynucleotide which contains the polymorphism, allowing (after binding) the two probes to be ligated together by an appropriate ligase enzyme. However the two probes will only bind (in a manner which allows ligation) to a polynucleotide that contains the polymorphism, and therefore the detection of the ligated product may be used to determine the presence of the polymorphism.

In one embodiment the probe is used in a heteroduplex analysis based system to detect polymorphisms. In such a system when the probe is bound to a polynucleotide sequence containing the polymorphism it forms a heteroduplex at the site where the polymorphism occurs (i.e. it does not form a double strand structure). Such a heteroduplex structure can be detected by the use of an enzyme that is single or double strand specific. Typically the probe is an RNA probe and the enzyme used is RNAse H that cleaves the heteroduplex region, thus allowing the polymorphism to be detected by means of the detection of the cleavage products.

The method may be based on fluorescent chemical cleavage mismatch analysis which is described for example in PCR Methods and Applications 3:268-71 (1994) and Proc. Natl. Acad. Sci. 85:4397-4401 (1998).

In one embodiment the polynucleotide agent is able to act as a primer for a PCR reaction only if it binds a polynucleotide containing the polymorphism (i.e. a sequence- or allele-specific PCR system). Thus a PCR product will only be produced if the polymorphism is present in the polynucleotide of the individual. Thus the presence of the polymorphism may be determined by the detection of the PCR product. Preferably the region of the primer which is complementary to the polymorphism is at or near the 3' end the primer. In one embodiment of this system the polynucleotide the agent will bind to the wild-type sequence but will not act as a primer for a PCR reaction.

The method may be an Restriction Fragment Length Polymorphism (RFLP) based system. This can be used if the presence of the polymorphism in the polynucleotide creates or destroys a restriction site that is recognized by a restriction enzyme. Thus treatment of a polynucleotide with such a polymorphism will lead to different products being produced compared to the corresponding wild-type sequence. Thus the detection of the presence of particular restriction digest products can be used to determine the presence of the polymorphism.

The presence of the polymorphism may be determined based on the change that the presence of the polymorphism makes to the mobility of the polynucleotide or protein during gel electrophoresis. In the case of a polynucleotide single-stranded conformation polymorphism (SSCP) analysis may be used. This measures the mobility of the single stranded polynucleotide on a denaturing gel compared to the corresponding wild-type polynucleotide, the detection of a difference in mobility indicating the presence of the polymorphism. Denaturing gradient gel electrophoresis (DGGE) is a similar system where the polynucleotide is electrophoresed through a gel with a denaturing gradient, a difference in mobility compared to the corresponding wild-type polynucleotide indicating the presence of the polymorphism.

The presence of the polymorphism may be determined using a fluorescent dye and quenching agent-based PCR assay such as the Taqman PCR detection system. In brief, this assay uses an allele specific primer comprising the sequence around, and including, the polymorphism. The specific primer is labeled with a fluorescent dye at its 5' end, a quenching agent at its 3' end and a 3' phosphate group preventing the addition of nucleotides to it. Normally the fluorescence of the dye is quenched by the quenching agent present in the same primer. The allele specific primer is used in conjunction with a second primer capable of hybridizing to either allele 5' of the polymorphism.

In the assay, when the allele comprising the polymorphism is present Taq DNA polymerase adds nucleotides to the nonspecific primer until it reaches the specific primer. It then releases polynucleotides, the fluorescent dye and quenching agent from the specific primer through its endonuclease activity. The fluorescent dye is therefore no longer in proximity to the quenching agent and fluoresces. In the presence of the allele which does not comprise the polymorphism the mismatch between the specific primer and template inhibits the endonuclease activity of Taq and the fluorescent dye in not released from the quenching agent. Therefore by measuring the fluorescence emitted the presence or absence of the polymorphism can be determined.

In another method of detecting the polymorphism a polynucleotide comprising the polymorphic region is sequenced across the region which contains the polymorphism to determine the presence of the polymorphism.

Accordingly, any of the following techniques may be utilized in the present methods for genotyping, as is known in the art.
- General: DNA sequencing, sequencing by hybridization;
- Scanning: PTT (Protein truncation technique), SSCP (single strand conformational analysis), DGGE (denaturing gradient gel electrophoresis), TGGE (temperature gradient gel electrophoresis), Cleavase, Heteroduplex analysis, CMC (chemical mismatch cleavage), enzymatic mismatch cleavage;
- Hybridization based: solid phase hybridization (dot blots, MASDA, reverse dot blots, oligonucleotide arrays (chips)); solution phase hybridization (Taqman, Molecular Beacons);
- Extension based: ARMS (Amplification Refractory Mutation System), ALEX (Amplification Refractory Mutation System Linear Extension) SBCE (Single Base Chain Extension)
- Incorporation based: Mini-sequencing, APEX; (Arrayed Primer Extension)
- Restriction enzyme based: RFLP (restriction fragment length polymorphism)
- Ligation based: OLA (Oligonucleotide Extension Assay)
- Other: Invader (Third Wave Technologies).

The present invention also provides for a predictive (patient care) test or test kit. Such a test will aid in disease management of IBS based on pre-determined associations between genotype and phenotypic response to 5HT ligands in treating gastrointestinal disease. Such a test could take two different formats:
- a molecular test which analyses DNA or RNA for the presence of pre-determined polymorphisms. An appropriate test kit may include one or more of the following reagents or instruments: a means to detect the binding of the agent to the polymorphism, an enzyme able to act on a polynucleotide (typically a polymerase or restriction enzyme), suitable buffers for enzyme reagents, PCR primers which bind to regions flanking the polymorphism, a positive or negative control (or both), a gel electrophoresis apparatus and a means to isolate DNA from a sample. The product may utilise one of the chip technologies as described by the current state of the art. The test kit would include printed or machine readable instructions setting forth the correlation between the presence of a specific polymorphism or genotype and the likelihood that a subject with IBS will respond favorably to therapy with a 5HT ligand.
- a biochemical test which analyses materials derived from the subject's body, including proteins or metabolites, that indicate the presence of a pre-determined polymorphism.. An appropriate test kit would comprise a molecule, aptamer, peptide or antibody (including an antibody fragment) that specifically binds to a predetermined polymorphic region (or a specific region flanking the polymorphism), or a binding agent as defined herein. The product may additionally comprise one or more additional reagents or instruments (as are known in the art). The test kit would also include printed or machine-readable instructions setting forth the correlation between the presence of a specific polymorphism or genotype and the likelihood that a subject with IBS will respond favorably to therapy with a 5HT ligand.

The invention provides a method for screening a subject diagnosed with IBS or another gastrointestinal disorder treatable by 5HT ligands, to determine the likelihood they will respond in a particular way to treatment with a 5HT ligand, more particularly a 5HT3 antagonist, and more particularly alosetron. The method comprises screening the subject for a polymorphism in the 5HTT gene that has previously been associated with a high or low incidence of a particular desirable therapeutic outcome (compared to the incidence in subjects with other genotypes), or associated with a high or low incidence of an undesired side effect (compared to the incidence in subjects with other genotypes). Subjects are mammalian, and preferably humans. Treatment of a subject with a 5HT ligand comprises administration of an effective amount of the pharmaceutical agent to a subject in need thereof. The dose of agent is determined according to methods known and accepted in the pharmaceutical arts, and can be determined by those skilled in the art. A suitable dosage range and plasma concentration for alosetron are provided in the disclosure of US Patent Number 5,360,800, the entire disclosure of which is hereby incorporated herein by reference.

### EXAMPLES

### Example 1: Assay of insertion/deletion polymorphism in 5HTT gene

Genetic samples were obtained from 219 female human subjects enrolled in clinical trials of alosetron for the treatment of IBS. Using PCR technology as is known in the art, an insertion/deletion genetic marker was assayed in the 5-hydroxytryptamine transporter gene (5HTT gene). The alleles were labeled as "del" (deletion) or "ins" (insertion) resulting in three possible genotypes (del/del; del/ins or ins/ins).

The insertion/deletion marker was in the 5' untranslated region of the 5HTT gene. The deletion polymorphism (allele "del") had SEQ ID NO:I; the insertion polymorphism (allele "ins") had SEQ ID NO:2 (insertion shown in bold typeface): The deleted segment comprised nucleotides 161- 204 of SEQ ID NO:2. PCR primer sequences are in underlined typeface.

The present 5HTT genotypes were approximately evenly distributed. Of the 219 subjects genotyped for the 5HTT marker, 71 (32.4%) were del/del 5HTT, 75 (34.2%) were del/ins 5HTT and 73 (33.3%) were ins/ins 5HTT.

The "del" allele represents a deletion of approximately 44 base pairs in the 5' untranslated region of the 5HTT gene. The del/del genotype results in a lower transcription efficiency, lower production of 5HTT, and reduced basal 5HT re-uptake (compared to the del/ins or ins/ins genotype).

### Example 2: Correlation of genotype and phenotype

The subjects' response to alosetron in the clinical trial setting was reviewed and correlated with genotype. In the double-blind, placebo controlled clinical trials, subjects received 12 weeks of treatment with either alosetron or a placebo. A favorable response to alosetron was when a subject reported relief of IBS symptoms during six weeks of the twelve week trial. The incidence of various other effects, including constipation, was also recorded.

The response of subjects to treatment with alosetron in the clinical trial was stratified according to genotype.

In each of the three 5HTT genotypes alosetron was more effective than placebo in producing relief (**Figure 1**). However, in the del/del genotype group (homozygous for the deletion polymorphism), an increased incidence of relief of IBS symptoms was seen (increased compared to other 5HTT genotypes). (**Figure 1**). Relief of IBS symptoms with alosetron was achieved in 68% of del/del subjects (21/31); 64% of del/ins subjects (21/33); and 58% of ins/ins subjects (22/38).

The occurrence of constipation during alosetron treatment in the clinical trial was stratified according to genotype. Alosetron treated subjects with the del/del genotype showed a reduced incidence of constipation compared to the del/ins and ins/ins 5HTT genotype groups (**Figure 2**) Constipation was reported in 21% of the total group of subjects receiving alosetron (n=102). In del/del subjects (n=31), 4 (13%) reported constipation; in del/ins subjects (n=33), 10 (30%) reported constipation; and in ins/ins subjects (n=38), 8 (21)% reported constipation (**Figure 2**).

Subjects with the del/del 5HTT genotype showed an increased incidence of favourable therapeutic response, with higher incidence of relief of IBS symptoms and lower incidence of constipation, when compared with subjects with del/ins and ins/ins 5HTT genotypes (**Figure 3**).

### EXAMPLE 3: Genotyping of Individuals for 5HTT polymorphisms

DNA samples are obtained from a population of subjects with gastrointestinal disease, and genomic DNA is extracted using standard procedures (automated extraction or using kit formats). The genotypes of the subjects, and any control individuals utilized, are determined for polymorphisms within the 5HTT gene sequence, using either PCR, PCR-RFLP, Taqman allelic discrimination assays, or any other suitable technique as is known in the art.

If a specific polymorphism resides in an amplification product that is of sufficient physical size (e.g., an insertion/deletion polymorphism of multiple bases), a simple size discrimination assay can be employed to determine the genotype of an individual. In this case, two primers are employed to specifically amplify the gene of interest in a region surrounding the site of the polymorphism. PCR amplification is carried out, generating products which differ in length, dependent on the genotype (insertion or deletion) they possess. When subjected to gel electrophoresis, the differently sized products are separated, visualized, and the specific genotypes interpreted directly.

PCR-RFLP (polymerase chain reaction - restriction fragment length polymorphism) assays may also be utilized as is known in the art to detect polymorphisms. For each polymorphic site, a PCR-RFLP assay employs two gene-specific primers to anneal to, and specifically amplify a segment of genomic DNA surrounding the polymorphic site of interest. Following PCR amplification, specific restriction endonuclease enzymes are employed to digest the PCR products produced. The enzyme utilized for an assay is selected due to its specific recognition sequence which it requires to bind to, and cleave the PCR product in the presence/absence of the polymorphism, yielding fragments diagnostic of the specific base present at the polymorphic site. Following cleavage by the restriction enzyme, gel electrophoresis is employed to separate and visualize the fragments produced.

Taqman assays, as are known in the art, may also be utilized to identify polymorphisms. For each polymorphic site the allelic discrimination assay uses two allele specific probes labeled with a different fluorescent dye at their 5' ends but with a common quenching agent at their 3' ends. Both probes have a 3' phosphate group so that Taq polymerase cannot add nucleotides to them. The allele specific probes comprising the sequence encompassing the polymorphic site and will differ only in the sequence at this site (this is not necessarily true, the allele-specific probes can be shifted relative to each other such that they are not identical in length or composition. However, where they cover the same DNA region they are identical apart from the polymorphic site of interest). The allele specific probes are only capable of hybridizing without mismatches to the appropriate site.

The allele specific probes are used in conjunction with two primers, one of which hybridizes to the template 5' of the two specific probes, whilst the other hybridizes to the template 3' of the two probes. If the allele corresponding to one of the specific probes is present, the specific probe will hybridize perfectly to the template. The Taq polymerase, extending the 5' primer, will then remove the nucleotides from the specific probe, releasing both the fluorescent dye and the quenching agent. This will result in an increase in the fluorescence from the dye no longer in close proximity to the quenching agent.

If the allele specific probe hybridizes to the other allele the mismatch at the polymorphic site will inhibit the 5' to 3' endonuclease activity of Taq and hence prevent release of the fluorescent dye.

The ABI7700 sequence detection system is used to measure the increase in the fluorescence from each specific dye at the end of the thermal cycling PCR directly in PCR reaction tubes. The information from the reactions is then analyzed. If an individual is homozygous for a particular allele only fluorescence corresponding to the dye from that specific probe will be released, but if the individual is heterozygous, then both dyes will fluoresce.

The genotypes of the individuals are then correlated with their phenotypic response to treatment with a 5HT ligand. Responses that vary among the genetic subpopulations are identified.

## Claims

1. A method of screening a subject suffering from a gastrointestinal disease that is treatable with a 5-hydroxytryptamine 3 (5HT3) antagonist, as an aid in predicting the subject's response to said treatment, comprising:
determining the genotype of said subject at a polymorphic insertion/deletion allelic site in the 5' non-coding region of the 5-hydroxytryptamine transporter (5HTT) gene, where a homozygous deletion (SEQ ID NO: 1) genotype indicates that the subject is more likely to have a favourable response to treatment with a 5HT3 antagonist, compared to a subject who is heterozygous or homozygous for the insertion allele (SEQ ID NO:2).

2. A method according to claim 1 where said subject suffers from Irritable Bowel Syndrome (IBS).

3. The method of claim 1 wherein the antagonist is selected from the group consisting of: Alosetron, granisetron, ondansetron.

## Patentansprüche

1. Verfahren zum Screenen einer Testperson, die an einer gastrointestinalen Krankheit leidet, die mit einem 5'-Hydroxytryptamin 3 (5HT3)-Antagonisten behandelbar ist, als Hilfe bei der Vorhersage der Reaktion der Testperson auf die Behandlung, umfassend:
Bestimmen des Genotyps der Testperson an einem polymorphen Insertions-/Deletionsallellocus in der 5'-nicht-codierenden Region des 5-Hydroxytryptamintransporter-(5HTT)-Gens, wobei ein homozygoter Deletions-(SEQ ID NR: 1)-Genotyp anzeigt, dass es wahrscheinlicher ist, dass die Testperson im Vergleich zu einer Testperson, die für das Insertionsallel (SEQ ID NR: 2) heterozygot oder homozygot ist, auf eine Behandlung mit einem 5HT3-Antagonisten eine günstige Reaktion aufweist.

2. Verfahren gemäß Anspruch 1, wobei die Testperson an Reizkolon (Irritable Bowel Syndrome, IBS) leidet.

3. Verfahren gemäß Anspruch 1, wobei der Antagonist ausgewählt ist aus der Gruppe bestehend aus: Alosetron, Granisetron, Ondansetron.

## Revendications

1. Méthode pour dépister un sujet souffrant d'une maladie gastro-intestinale qui peut être traitée avec un antagoniste de 5-hydroxytryptamine 3 (5HT3), comme auxiliaire dans la prévision de la réponse du sujet audit traitement, comprenant :
la détermination du génotype dudit sujet à un site allélique d'insertion/délétion polymorphe dans la région non codante 5' du gène transporteur de 5-hydroxytryptamine (5HTT), un génotype de délétion homozygote (SEQ ID N°1) indiquant que le sujet est plus apte à présenter une réponse favorable au traitement avec un antagoniste de 5HT3 comparativement à un sujet qui est hétérozygote ou homozygote pour l'allèle d'insertion (SEQ ID N°2).

2. Méthode suivant la revendication 1, dans laquelle ledit sujet souffre du syndrome du côlon irritable (IBS).

3. Méthode suivant la revendication 1, dans laquelle l'antagoniste est choisi dans le groupe consistant en : l'alosétron, le granisétron, l'ondansétron.


### SEQUENCE LISTING

<110> Manasco. Penelope
   Mosteller. Michael
   Kong, Ning
<120> 5-Hydroxytryptamine Transporter Gene Polymorphisms
<130> PU3932W0
<160> 2
<170> PatentIn version 3.0
<210> 1
   <211> 484
   <212> DNA
   <213> Homo sapiens
<400> 1
   ggcgttgccg ctctgaatgc cagccctaac ccctaatgtc cctactgcag cctcccagca     60
   tcccccctgc aacctcccag caactccctg tacccctcct aggatcgctc ctgcatcccc    120
   cattatcccc cccttcactc ctcgcggcat cccccctgca cccccagcat cccccctgca    180
   gcccttccag catccccctg cacctctccc aggatctccc ctgcaacccc cattatcccc    240
   cctgcacccc tcgcagtatc ccccctgcac cccccagcat ccccccatgc acccccggca    300
   tcccccctgc acccctccag cattctcctt gcaccctacc agtattcccc cgcatcccgg    360
   cctccaagcc tcccgcccac cttgcggtcc ccgccctggc gtctaggtgg caccagaatc    420
   ccgcgcggac tccacccgct gggagctgcc ctcgcttgcc cgtggttgtc cagctcagtc    480
   cctc                                                                  484
<210> 2
   <211> 528
   <212> DNA
   <213> Homo sapiens
<400> 2
   ggcgttgccg ctctgaatgc cagccctaac ccctaatgtc cctactgcag cctcccagca    60
   tcccccctgc aacctcccag caactccctg tacccctcct aggatcgctc ctgcatcccc   120
   cattatcccc cccttcactc ctcgcggcat cccccctgca ccccccagca tcccccctgc   180
   agccccccca gcatctcccc tgcaccccca gcatcccccc tgcagccctt ccagcatccc   240
   cctgcacctc tcccaggatc tcccctgcaa cccccattat cccccctgca cccctcgcag   300
   tatcccccct gcacccccca gcatcccccc atgcaccccc ggcatccccc ctgcacccct   360
   ccagcattct ccttgcaccc taccagtatt cccccgcatc ccggcctcca agcctcccgc   420
   ccaccttgcg gtccccgccc tggcgtctag gtggcaccag aatcccgcgc ggactccacc   480
   cgctgggagc tgccctcgct tgcccgtggt tgtccagctc agtccctc                528